# EUROPÄISCHE PATENTSCHRIFT

(11) **EP 0 607 467 B1**
(45) Veröffentlichungstag und Bekanntmachung des Hinweises auf die Patenterteilung: **02.06.1999**
(21) Anmeldenummer: 92810938.8
(22) Anmeldetag: 01.12.1992
(51) Int. Cl.: A61K 31/19, A61K 9/16, A61K 9/20, A61K 9/50

(54) **S(+)-Ibuprofen enthaltende Arzneimittel**
Compositions containing S(+)-Ibuprofen
Compositions pharmaceutiques à base de S(+)-Ibuprofène

(43) Veröffentlichungstag der Anmeldung: 27.07.1994
(73) Patentinhaber: SPIRIG AG PHARMAZEUTISCHE PRÄPARATE, CH-4622 Egerkingen (CH)
(72) Erfinder: Birrenbach, Gerd, CH-4616 Kappel (SO) (CH); Juch, Rolf-Dieter, CH-4612 Wangen b. Olten (CH)
(74) Vertreter: Braun, André, jr.

(56) Entgegenhaltungen:
- EP-A- 0 478 838
- WO-A-92/20334

## Beschreibung

Ibuprofen, 2-(4-isobutylphenyl)-propionsäure, ist bekannt als nicht-steroidales Antiphlogistikum (NSA), das eine gute anti-entzündliche, analgetische und antipyretische Wirkung zeigt. Es wird vor allem in der Therapie der chronischen Polyarthritis und der Osteoarthritis eingesetzt. Gute Wirkung zeigt Ibuprofen auch bei der Behandlung von Dysmenorrhö, von Gicht und von milden bis mässigen Schmerzzuständen. Ibuprofen wurde 1969 zum ersten Mal in der Klinik eingesetzt und umfangreiche Erfahrungen über seine Anwendung liegen vor.

Als Arylpropionsäure-Derivat enthält Ibuprofen ein asymmetrisches Kohlenstoffatom. Bei allen im Handel erhältlichen Präparaten liegt Ibuprofen als Racemat vor, d.h. als eine 50:50 Mischung der beiden Enantiomere R(-)- und S(+)-Ibuprofen. Die pharmakologische Aktivität von Ibuprofen liegt, wie bei den meisten NSA, in einer Hemmung der Prostaglandin-Synthese durch Bindung an die Zyklooxygenase-Untereinheit des Enzyms Prostaglandin-Synthease. Dieser für die Therapie relevante Wirkungsmechanismus wurde in vitro nur bei dem S(+)-Enantiomer gefunden. Jedoch konnte in vivo diese Enantioselektivität nicht bestätigt werden. Diese Beobachtung erklärt sich dadurch, dass in vivo das R(-)-Enantiomer in das pharmakodynamisch aktive S(+)-Enantiomer umgewandelt wird. Diese Umwandlung ist ein enzymatischer Prozess, an dem nur das R(-)-Enantiomer beteiligt ist, d.h., er verläuft stereoselektiv und unidirektionell. Nachgewiesen wurde er bei fast allen Labortierspezies und beim Menschen.

In den letzten Jahren hat sich die Meinung verbreitet, dass, wenn möglich, chirale Wirkstoffe in die entsprechenden Enantiomere getrennt werden sollten. Die relativen pharmakodynamischen Aktivitäten der Enantiomere und deren mögliche pharmakokinetischen und metabolischen Unterschiede wurden untersucht. Dabei wurde erkannt, dass sogar molekulare Wechselwirkungen am Wirkungsort und/oder während des Transports durch biologische Membranen möglich sind. Im speziellen Fall von Ibuprofen könnte man annehmen, dass die schnelle Umwandlung des R(-)-Enantiomers in die aktive S(+)-Form die Anwendung des Racemats rechtfertigt. Eine detaillierte Analyse der relevanten Literatur zeigt jedoch, dass die Verwendung des S(+)-Enantiomers allein deutliche Vorteile mit sich bringt. Diese neueren Ergebnisse können wie folgt zusammengefasst werden:

Die Umwandlung von R(-) in S(+) ist unvollständig und zeigt eine erhebliche Variabilität. Daher ist die effektive Dosis an S(+)-Ibuprofen, die der Körper eines Patienten aufnimmt, im voraus kaum exakt zu bestimmen. Die Bindung an die Serumproteine, die bei den NSA eine grosse Rolle spielt, ist stereoselektiv. Dabei zeigen die R(-)- und die S(+)-Form eine Kompetitivität für die Bindungsstelle. Der gebundene Anteil ist deshalb von den relativen Mengen der beiden Enantiomere im Plasma abhängig. Dies hat signifikante Konsequenzen auf die Diffusion des Wirkstoffs in therapeutisch relevante Kompartimente, wie z.B. die Synovialflüssigkeit. Die multi-enzymatische Reaktion, die zur Umwandlung des R(-)-Ibuprofens führt, beginnt mit der selektiven Bindung dieses R(-)-Enantiomers an das Coenzym A. Dies führt zur Bildung von symmetrischen Thioestern, die vom Organismus als "Fettsäureester" erkannt werden und in das Fettgewebe eingebaut werden. Daraus werden diese sehr langsam ausgeschieden. Bei chronischer Verabreichung des Racemats findet demnach eine Akkumulation des R(-)-Enantiomers im Fettgewebe statt. Obwohl bis jetzt keine toxischen Wirkungen beobachtet wurden, die in direkte Kombination zu dieser Akkumulation zu bringen sind, stellen das verlängerte Vorhandensein des Wirkstoffs im Organismus und die verlangsamte Ausscheidung unerwünschte Eigenschaften dar, die toxikologische Bedeutung erlangen könnten, z.B. bei Hypersensibilität. Ein Austausch des Racemats durch das aktive S(+)-Enantiomer erscheint demnach begründet.

Es wurde gezeigt, dass nach oraler Verabreichung die Absorptionsgeschwindigkeit aus dem Magen-Darm-Trakt einen direkten Einfluss auf das Ausmass der Umwandlung hat. Dies trägt zur oben genannten Variabilität bei und hat ausserdem direkte Konsequenzen für die Bioverfügbarkeit und die Bioäquivalenz verschiedener galenischer Formulierungen des Racemats. Dies gilt insbesondere bei Arzneimittelformen mit unterschiedlichen Absorptionsraten, wie z.B. bei Retard-Formen gegenüber schnell freisetzenden Formen. Verschiedene, in den letzten Jahren veröffentlichte Untersuchungen belegen, dass nach aktuellen Kriterien als bioäquivalent betrachtete Racemat-Präparate nicht mehr bioäquivalent sind, wenn die Enantiomere getrennt betrachtet werden. Bei Verabreichung des S(+)-Enantiomers tragen diese Erkenntnisse, zusammen mit der einfachen Pharmakokinetik und der besseren Einstellmöglichkeiten der Patienten, entscheidend zur Sicherheit der chronischen Therapie bei.

In Untersuchungen an Tieren, Probanden und Patienten zeigte das S(+)-Enantiomer in niedrigerer Dosierung die gleiche therapeutische Wirkung wie das Racemat. An Rhesusaffen erwies sich S(+)-Ibuprofen als stärker analgetisch wirksam. An einem Hautentzündungsmodell an gesunden Probanden war die Dosiswirkungskurve von S(+)-Ibuprofen gegenüber derjenigen des Racemats in Richtung geringerer Dosierung verschoben. An Arthritis-Patienten wurde eine gute therapeutische Wirkung bei einer täglichen Dosis ermittelt, die deutlich unter der üblichen Racemat-Dosierung lag. Diese Erkenntnisse zeigen, dass es möglich ist, mit niedrigerer Dosierung die gleiche therapeutische Wirksamkeit zu erreichen. Auch das trägt zur Sicherheit der Patienten bei.

Wohl auf Grund dieser Erkenntnisse wird im EP-B-0 267 321 ein Ibuprofen enthaltendes Arzneimittel beschrieben, in dem Ibuprofen nur als S(+)-Ibuprofen mit üblichen Träger- und Verbindungsmitteln enthalten ist, wobei das Arzneimittel dadurch gekennzeichnet ist, dass es entweder in Retard-Form oder aber auch in Kombination mit nicht retardiertem S(+)-Ibuprofen vorliegen kann. Die Retard-Form als kennzeichnendes Merkmal wurde deshalb gewählt, weil es bisher nicht möglich war, die Tagesdosen des Racemats von 1200 bis 2400 mg in den voluminösen Retardformen zur Verfügung zu stellen. Erst die durch Verwendung des S(+)-Enantiomeren mögliche Reduzierung der Tagesdosen liess die durch die Hilfsstoffe, Coatings u.a. bedingten voluminöseren Retardformen für den Patienten applikabel werden. Die Formulierung zu Tabletten, Dragees und andern für die Retardierung geeigneten Retardierungsformulierungen erfolgt dabei in üblicher Weise, wobei die bekannten Träger- und Hilfsstoffe verwendet werden. Die aus der Galenik bekannten Formulierungen können auch bei dieser Erfindung angewendet werden.

Für Analgetika von grosser Bedeutung ist das schnelle Einsetzen von deren Wirkung. In der US-Patentschrift 4,851,444 wird beschrieben, dass die analgetische Wirkung des S(+)-Ibuprofens im Verhältnis zu gleichen Mengen des Racemats schneller einsetzt und verstärkt wirkt. Beansprucht wird denn auch ein Verfahren zur schnelleren und verstärkten analgetischen Wirkung bei Menschen, das darin besteht, dass man eine geeignete Menge S(+)-Ibuprofen appliziert, das im wesentlichen frei von R(-)-Ibuprofen ist. Die geeigneten Formulierungen dazu können wiederum dem Stand der Technik entnommen werden. Da bei Verwendung des Racemats nur ein Teil des R(-)-Enantiomers in vivo in das aktive S(+)-Enantiomer übergeführt wird, liegt es auf der Hand, dass bei Verwendung gleicher Mengen des S(+)-Enantiomers und des Racemats das ausschliesslich das aktive Prinzip darstellende S(+)-Enantiomer eine verstärkte und schneller einsetzende Wirkung aufweist.

Wie in EP-A-0 288 732 beschrieben, kann man zur Verbesserung des Löslichkeitsverhalten von kugelförmigen ACE-Inhibitoren, Beta-Blockern, Ca-Kanal-Blockern und andern Pharmazeutika in einer Menge von 3-60 % organische Säuren in einer Menge von 5-50 % zugeben. In der EP-A-0 308 665 wird zur Erreichung des gleichen Ziels vorgeschlagen, zur schlecht wasserlöslichen 5-Aminosalicylsäure basische Hilfsstoffe im Bereich von 0,5-3-molar, bezogen auf die 5-Aminosalicylsäure, zur Bildung eines wasserlöslichen Salzes zuzugeben. Bei Verwendung dieser beiden technischen Lehren entstehen zwangsläufig wieder voluminöse Verabreichungsformen, da man wegen des Anstrebens einer kompletten Salzbildung mit molaren Äquivalenzen rechnen muss.

Ziel der Erfindung war es demnach, schnell freisetzende S(+)-Ibuprofen-Arzneimittel mit möglichst geringen Mengen an Hilfsstoffen zur Verfügung zu stellen. In der ersten Stufe wurden dazu S(+)-Ibuprofen-haltige Pellets aufgebaut.

Versuche in dieser Richtung schlugen vorerst fehl. Mit herkömmlichen Pelletierhilfsmitteln, wie Avicel, Lactose, Primojel und HPMC in diversen Kombinationen mit einem Wirkstoffgehalt zwischen 90 und 94 % lösten sich die Pellets praktisch gar nicht oder nur sehr schlecht im künstlichen Darmsaft. Es wurden auch Tenside zugegeben, um die Benetzung zu verbessern, ebenso auch zusätzlich PEG, um durch diesen hydrophilen Stoff die Wasserlöslichkeit zu fördern. Dabei konnte keine Beschleunigung der Freisetzung des Wirkstoffs im künstlichen Darmsaft festgestellt werden (vgl. Vergleichsbeispiele A.-F.).

Selbst durch Erhöhung der Pelletierhilfsstoffe auf 25 bis 30 % konnte keine Verbesserung der Wirkstofffreisetzung aus den Pellets beobachtet werden. Zudem wurde dadurch natürlich der Wirkstoffgehalt der Pellets, entgegen der Aufgabe der Erfindung, entsprechend reduziert. Schluckbare Arzneiformen mit bis zu 600 mg Wirkstoff wären mit derartigen Pellets - sei es abgefüllt in Kapseln oder aber verpresst zu Tabletten - nicht realisierbar.

Es wurde nun überraschend gefunden, dass im wesentlichen in Abwesenheit von Calciumsalzen durch Zugabe von geringen, nicht äquimolaren Mengen an Natriumcarbonat, Kaliumcarbonat und Dinatriumhydrogenphosphat oder Gemische davon Pellets mit hoher Konzentration an S(+)-Ibuprofen gebildet werden können, die den Wirkstoff gemäss USP XXII (Phosphatpuffer, pH 7,2) sehr schnell freisetzen.

Die Pellets enthalten dabei 90,0-99,0 Gew.-% S(+)-Ibuprofen, und 0,1-10,0 Gew.-% an Natriumcarbonat, Kaliumcarbonat und Dinatriumhydrogenphosphat.

Vorzugsweise enthalten die Pellets 96,0-98,0 Gew.-%, insbesondere 97 Gew.-%, S(+)-Ibuprofen und 1,0-3,0 Gew.-%, insbesondere 2,0 Gew-%, an Natriumcarbonat, Kaliumcarbonat und Dinatriumhydrogenphosphat.

Die Pellets können 0,1-5,0, insbesondere 2,0 Gew.-%, mindestens eines Hilfsstoffs, wie mikrokristalline Cellulose, Lactose, Hydroxypropylmethylcellulose (HPMC) und/oder vorzugsweise Siliciumdioxid enthalten.

Eine bevorzugte Ausführungsform der Pellets enthält 90 Gew.-% bis 99 Gew.-%, vorzugsweise 97 Gew.-%, S(+)-Ibuprofen, 0,1 Gew.-% bis 5,0 Gew.-%, vorzugsweise 1,0 Gew.-%, Siliciumdioxid und 0,1 Gew.-% bis 10,0 Gew.-%, vorzugsweise 2,0 Gew.-%, Natriumcarbonat.

Nebst Verbesserung des Löslichkeitsverhaltens bewirkt die Zugabe eines erfindungsgemässen basischen anorganischen Salzes während des Pelletiervorgangs ein schwaches Anlösen des S(+)-Ibuprofen, worauf dieses leicht klebrig wird, so dass die Verwendung von zusätzlichen Bindemitteln sich erübrigt.

Schonend hergestellt werden können die Pellets insbesondere in einem Rotorprozessor, wie er z.B. von Niro-Aeromatic, Bubendorf, Schweiz, vertrieben wird, oder einem schnell laufenden Mischer, wie er z.B. einem Diosnamischer von Dierks und Söhne, Osnabrück, Deutschland, vertrieben wird.

Durch Besprühen der mindestens einen Hilfsstoff enthaltenden S(+)-Ibuprofen-haltigen Mischung im Rotorprozessor oder Diosna-Mischer mit einem basischen anorganischen Salz in wässriger Lösung oder einer verdünnten Alkalilauge kann man Pellets herstellen. Die gebildeten Pellets werden getrocknet und gegebenenfalls fraktioniert. Auf diese Art erhält man ausgezeichnet gerundete Pellets von grosser Kompaktheit, von denen sich 90% innerhalb von 20 Minuten unter Bedingungen der USP XXII, Seite 1784/85 (Phosphatpuffer, pH 7,2) freisetzen.

Die Pellets können gegebenenfalls mit einem Coating versehen werden. Das Coating dient dem besseren Handling der Pellets beim Einfüllen in die Hartgelatine-Kapseln und/oder der verzögerten Wirkstofffreisetzung. Insbesondere können die gecoateten Pellets mit geringen Mengen an geeigneten Hilfsstoffen direkt zu Tabletten verpresst werden, was mit dem reinen Wirkstoff allein bisher nur unter Zuhilfenahme von grossen Mengen an Tablettierhilfsstoffen (wegen der grossen Klebrigkeit des Wirkstoffs bei üblichen Verarbeitungstemperaturen) und gegebenenfalls technisch aufwendigen Verfahrensschritten (z.B. Presstemperatur <10°C wegen des tiefen Schmelzpunktes des S(+)-Ibuprofen) möglich war. Zudem weisen die erfindungsgemässen Tabletten den Vorteil auf, dass der extrem seifig-bittere und nachhaltige Geschmack von S(+)-Ibuprofen maskiert wird. Die Tabletten können so ohne Widerwillen und Geschmacksirritation eingenommen und geschluckt werden, ohne dass ein zusätzliches Schutzcoating - wie sonst bei diesen Tabletten üblich - erfolgen müsste. Mithin kann der extrem schwierig zu verarbeitende Wirkstoff S(+)-Ibuprofen über die beschriebene Pelletierung in einem bisher nicht ausführbaren einfachen Tablettierprozess zu kompakten, schnell freisetzenden Tabletten, die zudem noch vorteilhaft geschmacksmaskiert sind, verarbeitet werden.

Geeignete Schutzcoatings der Pellets können 0,1-10,0 Gew.-% (wobei die Gew.-% jeweils immer auf das ungecoatete Pelletgewicht bezogen sind), vorzugsweise 2,0 Gew.-%, Hydroxypropylmethylcellulose (HPMC), Hydroxypropylcellulose (HPC), Polyvinylpyrrolidon (PVP), Polyvinylacetat (PVA), Methylcellulose (MG) oder Gemische davon oder 0,1-20 Gew.-%, vorzugsweise 10 Gew.-%, einer Methacrylsäure und/oder eines Ethylacrylats (in Suspension, 30% Feststoffanteil), wie Eudragit® L 30 D, enthalten.

Geeignete magensaftresistente Coatings können 0,1-60,0 Gew.-%, vorzugsweise 25,0 Gew.-%, einer Methacrylsäure und/oder eines Ethylacrylats (in Suspension, 30% Feststoffanteil), wie Eudragit® L 30 D, Hydroxypropylmethylcellulosephthalat (HPMCP) und/oder Celluloseacetatphthalat enthalten.

Geeignete retardierende Coatings können 0,1-50,0 Gew.-%, vorzugsweise 20,0 Gew.-%, eines Ethylacrylats und/oder Methylmethacrylats (Suspension, 30% Feststoffanteil), wie Eudragit® NE 30 D und/oder Eudragit® RS 30 D und/oder Eudragit® RL 30 D, und/oder Ethylcellulose enthalten.

Alle Coatings werden mit den üblicherweise erforderlichen und bekannten Hilfsstoffen und den entsprechenden technischen Mitteln aufgetragen. Dabei ist es für den Einsatz bei Pharmazeutika und zum Schutz der Umwelt sinnvoll, sich bei sämtlichen Arbeitsprozessen auf rein wässrige Arbeitsmethoden zu stützen, wie sie heute schon zum Stand der Technik gehören.

Die verschiedenen Arten des Coatings können alleine oder in irgendeiner Kombination miteinander benützt werden. So enthält ein bevorzugtes Schutzcoating eine Kombination aus HPMC oder Eudragit® L 30 D und Talcum, Titandioxid und/oder Siliciumdioxid und gegebenenfalls Antischaumemulsion, Diethylphthalat und/oder Polyethylenglykol (PEG), ein bevorzugtes magensaftresistentes Coating eine Kombination aus Eudragit® L 30 D, Diethylphthalat, Siliciumdioxid und gegebenenfalls Antischaumemulsion SE2 und ein bevorzugtes retardierendes Coating Eudragit® RS 30 D, Talcum, Diethylphthalat und gegebenenfalls Antischaumemulsion SE2.

Die Hilfsstoffe in einem bevorzugten Schutzcoating liegen vorzugsweise als Kombination in den folgenden Mengen vor:
im Falle von HPMC als Polymer: 0,1-8,0 Gew.-%, insbesondere 2,0 Gew.-%, Talcum; im Falle von Eudragit® L 30 D als Polymer: 0,1-8,0 Gew.-%, insbesondere 0,4 Gew.-%, Siliciumdioxid, 0,1-8,0 Gew.-%, insbesondere 0,4 Gew.-%, Titandioxid, 0,1-8,0 Gew.-%, insbesondere 0,2 Gew.-%, PEG als Weichmacher, und gegebenenfalls 0,01-0,5 Gew.-%, insbesondere 0,04 Gew. -%, Antischaumemulsion SE2.

Die Hilfsstoffe in einem bevorzugten magensaftresistenten Coating liegen vorzugsweise als Kombination in den folgenden Mengen vor:
0,1-8,0 Gew.-%, insbesondere 3,0 Gew.-%, Siliciumdioxid, 0,1-8,0 Gew.-%, insbesondere 1,0 Gew.-%, Diethylphthalat als Weichmacher, und gegebenenfalls 0,01-0,5 Gew.-%, insbesondere 0,04 Gew.-%, Antischaumemulsion SE2.

Die Hilfsstoffe in einem bevorzugten retardierenden Coating liegen vorzugsweise als Kombination in den folgenden Mengen vor:
0,1-8,0 Gew.-%, insbesondere 3,0 Gew.-%, Talcum, 0,1-8,0 Gew.-%, insbesondere 1,0 Gew.-%, Diethylphthalat als Weichmacher, und gegebenenfalls 0,01-0,5 Gew.-%, insbesondere 0,04 Gew.-%, Antischaumemulsion SE2.

Die gecoateten Pellets können mittels herkömmlicher Verfahren zu Tabletten verpresst werden, wobei diese auf 400 mg S(+)-Ibuprofen 73-410 mg, vorzugsweise 240-260 mg, besonders bevorzugt 250 mg, mindestens eines Tablettierhilfsmittels enthalten.

Als Tablettierhilfsmittel kommen insbesondere Mikrokristalline Cellulose, Calciumhydrogenphosphat, wie Emcompress, Lactose, Stärke, wie Maisstärke, Crosscarmellose Natrium, wie Ac-Di-Sol, Natriumstärkeglykolat, wie Primojel, PVP, HPMC, Magnesiumstearat, gehärtetes Rizinusöl, wie Cutina HR, Siliciumdioxid, wie Aerosil 200, Kaliumchlorid, Calciumchlorid, Natriumchlorid, Natriumcitrat, Kaliumadipat, Calciumlactat, und/oder Kaliumcitrat oder Gemische davon in Frage.

Ein bevorzugtes Gemisch an Tablettierhilfsmitteln enthält eine Kombination von Mikrokristalline Cellulose, Maisstärke, PVP, Magnesiumstearat, Siliciumdioxid und Kaliumchlorid.

Die Tabletten können 50-800 mg, vorzugsweise 100-600 mg, besonders bevorzugt 400 mg, S(+)-Ibuprofen, enthalten. Eine 400 mg S(+)-Ibuprofen enthaltende Tablette enthält als Tablettierhilfsmittel vorzugsweise 60 mg bis 200 mg, bevorzugt 160 mg, Mikrokristalline Cellulose, 10 mg bis 80 mg, vorzugsweise 40 mg Maisstärke, 1 mg bis 40 mg, vorzugsweise 14 mg, PVP, 1 mg bis 20 mg, vorzugsweise 8 mg Magnesiumstearat, 1 mg bis 30 mg, vorzugsweise 14 mg, Siliciumdioxid, 0,1 mg bis 40 mg, vorzugsweise 15 mg, Kaliumchlorid.

Die in vitro Zerfallszeit einer erfindungsgemässen Tablette ist dabei nach USP XXII kleiner als 2 Minuten und eine erfindungsgemässe Tablette weist eine Wirkstofffreisetzung nach USP XXII von über 90 % freigesetztem S(+)-Ibuprofen innerhalb von 20 Minuten (USP XXII, S. 1784/85, Phosphatpuffer, pH 7,2) auf.

Die gegebenenfalls gecoateten Pellets können auch in Kapseln abgefüllt werden. Durch den hohen Wirkstoffanteil des S(+)-Enantiomeren in Bezug auf die Hilfsstoffe können beispielsweise für 400 mg S(+)-Ibuprofen Kapseln der Grösse 0 verwendet werden, die noch mit bester Akzeptanz geschluckt werden können.

Durch Applikation der erfindungsgemässen Pellets bei Mensch und Tier kann im Verhältnis zum Racemat für äquivalente Plasmaspiegel mindestens eine Menge von 25% Wirkstoff eingespart werden.

In den folgenden Vergleichsbeispielen A-F werden nicht zum Erfolg führende Versuche der Pelletierung mit herkömmlichen Hilfsstoffen beschrieben, während in den Beispielen 1-11 die Erfindung näher erläutert wird, wobei - soweit nichts anderes angegeben ist - die Zahlenangaben in den Vergleichsbeispielen und in den Beispielen immer Gew.-% bedeuten.

### Vergleichsbeispiel A

| | |
|---|---|
| S(+)-Ibuprofen | 97,35 |
| Metolose 90SH 30000 (=Hydroxypropylmethylcellulose) | 0,15 |
| PEG 4000 | 2,00 |
| Tween® 80 | 0,50 |
| Aqua purificata | q.s. |

Der Wirkstoff und die Hilfsmittel werden gemischt. Das wasserlösliche PEG und das Tensid Tween® werden zur besseren Auflösung von S(+)-Ibuprofen beigemischt. Diese Mischung wird im Diosnamischer oder im Rotorprozessor auf übliche Weise solange mit Aqua purificata besprüht, bis sich Pellets der gewünschten Grösse bilden. Die Pellets werden dann getrocknet und mit dem Taumelsieb fraktioniert.

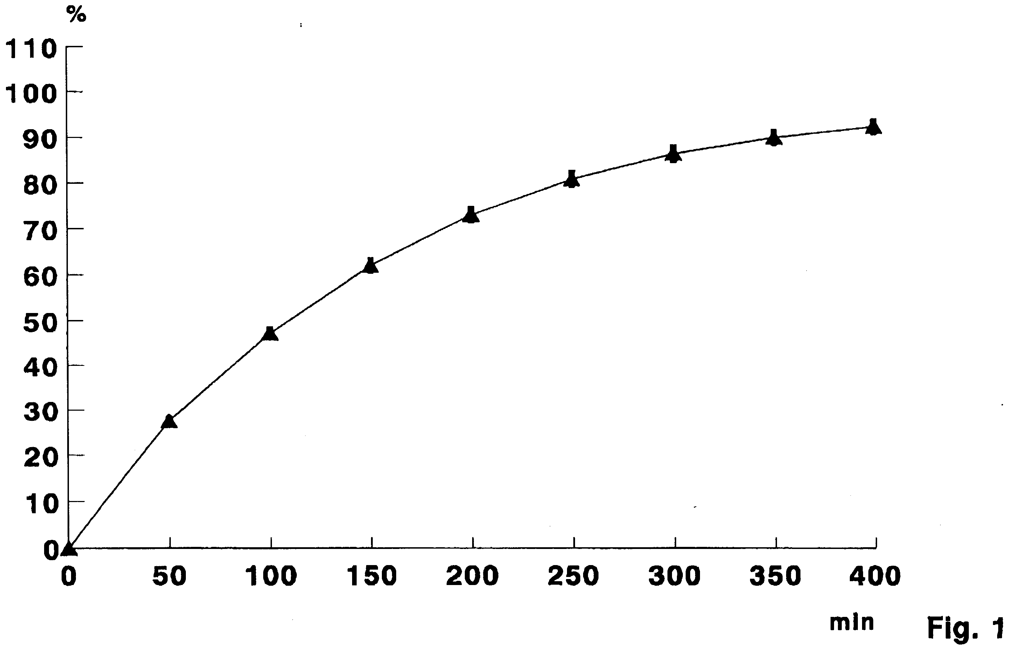

Die Resultate der in vitro Freisetzung der Pellets gemäss USP XXII (Phosphatpuffer, pH 7,2) in Fig. 1 zeigen das ungenügende Auflösungsverhalten dieser Pellets.

### Vergleichsbeispiel B

| | |
|---|---|
| S(+)-Ibuprofen | 95,00 |
| Mikrokristalline Cellulose | 1,5 |
| Pharmacoat 606 (=HPMC) | 1,00 |
| PEG 4000 | 2,0 |
| Tween® 80 | 0,5 |
| Aqua purificata | q.s. |

Der Wirkstoff und die Hilfsmittel werden gemischt. Das wasserlösliche PEG und das Tensid Tween® werden zur besseren Auflösung von S(+)-Ibuprofen beigemischt. Diese Mischung wird im Diosnamischer oder im Rotorprozessor auf übliche Weise solange mit Aqua purificata besprüht, bis sich Pellets der gewünschten Grösse bilden. Die Pellets werden dann getrocknet und mit dem Taumelsieb fraktioniert.

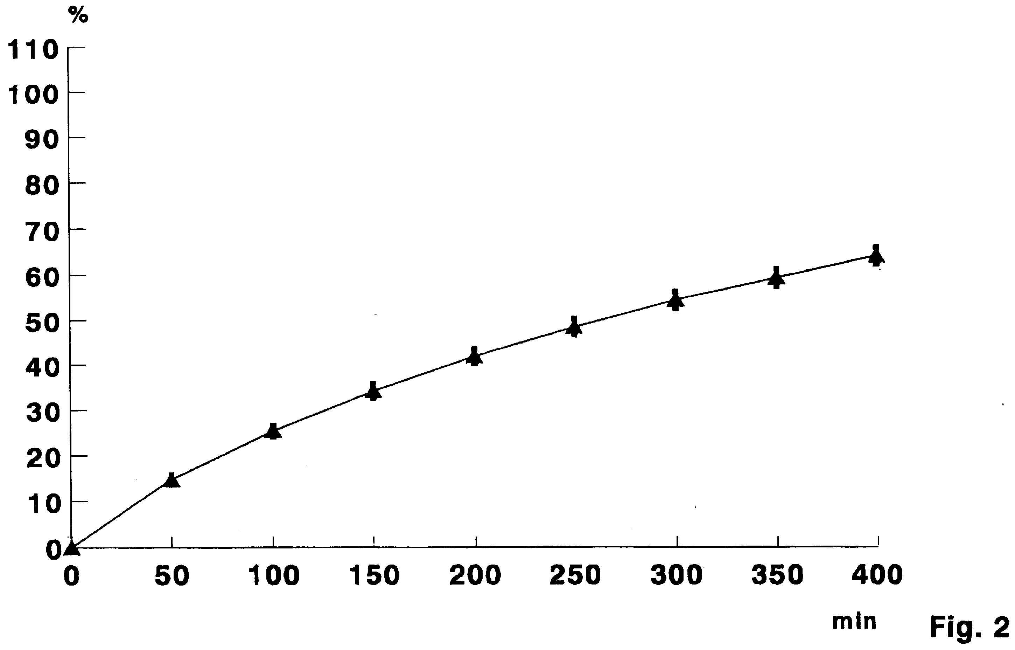

Die Resultate der in vitro Freisetzung der Pellets gemäss USP XXII (Phosphatpuffer, pH 7,2) in Fig. 2 zeigen das ungenügende Auflösungsverhalten dieser Pellets.

### Vergleichsbeispiel C

| | |
|---|---|
| S(+)-Ibuprofen | 90,00 |
| Mikrokristalline Cellulose | 4,5 |
| Pharmacoat 606 | 1,00 |
| PEG 4000 | 4,0 |
| Tween® 80 | 0,5 |
| Aqua purificata | q.s. |

Der Wirkstoff und die Hilfsmittel werden gemischt. Das wasserlösliche PEG und das Tensid Tween® werden zur besseren Auflösung von S(+)-Ibuprofen beigemischt. Diese Mischung wird im Diosnamischer oder im Rotorprozessor auf übliche Weise solange mit Aqua purificata besprüht, bis sich Pellets der gewünschten Grösse bilden. Die Pellets werden dann getrocknet und mit dem Taumelsieb fraktioniert.

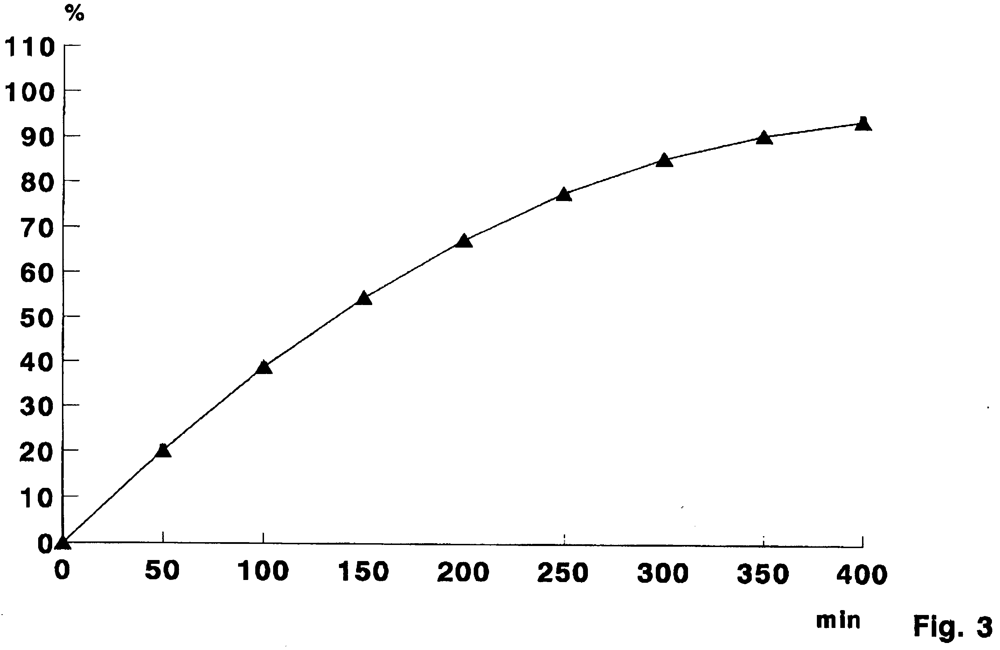

Die Resultate der in vitro Freisetzung der Pellets gemäss USP XXII (Phosphatpuffer, pH 7,2) in Fig. 3 zeigen das ungenügende Auflösungsverhalten dieser Pellets.

### Vergleichsbeispiel D

| | |
|---|---|
| S(+)-Ibuprofen | 70,00 |
| Mikrokristalline Cellulose | 15,00 |
| Lactose | 15,00 |
| Aqua purificata | q.s. |

Der Wirkstoff und die Hilfsmittel werden gemischt. Diese Mischung wird im Diosnamischer oder im Rotorprozessor auf übliche Weise solange mit Aqua purificata besprüht, bis sich Pellets der gewünschten Grösse bilden. Die Pellets werden dann getrocknet und mit dem Taumelsieb fraktioniert.

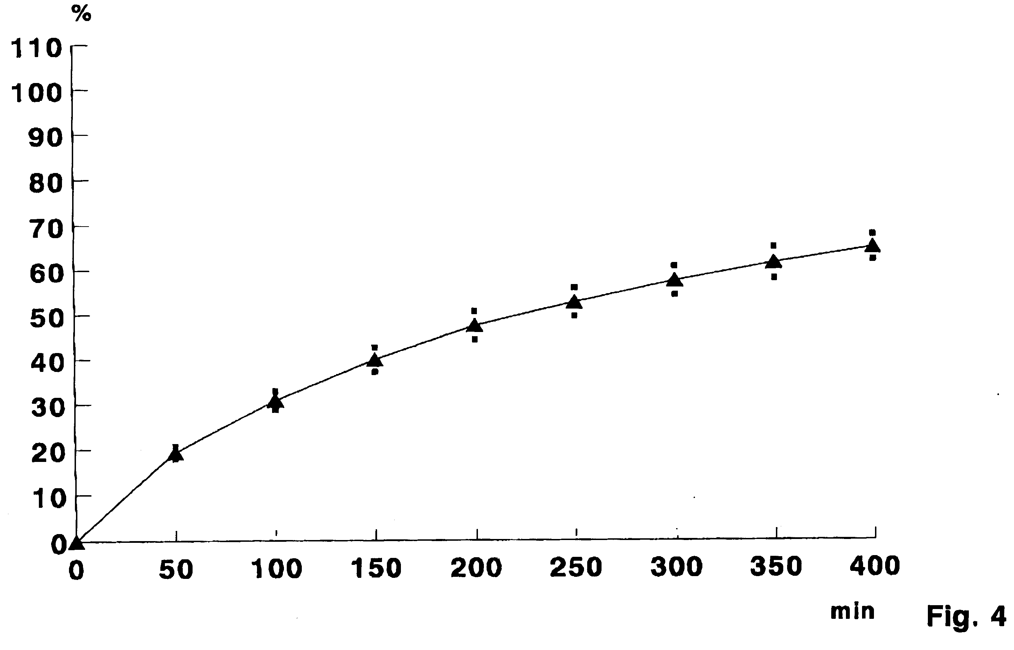

Die Resultate der in vitro Freisetzung der Pellets gemäss USP XXII (Phosphatpuffer, pH 7,2) in Fig. 4 zeigen das ungenügende Auflösungsverhalten dieser Pellets, obwohl der Anteil an Hilfsstoffen auf 30 % erhöht wurde.

### Vergleichsbeispiel E

| | |
|---|---|
| S(+)-Ibuprofen | 75,00 |
| Lactose | 15,00 |
| PEG 4000 | 10,00 |
| Aqua purificata | q.s. |

Der Wirkstoff und die Hilfsmittel werden gemischt. Diese Mischung wird im Diosnamischer oder im Rotorprozessor auf übliche Weise solange mit Aqua purificata besprüht, bis sich Pellets der gewünschten Grösse bilden. Die Pellets werden dann getrocknet und mit dem Taumelsieb fraktioniert.

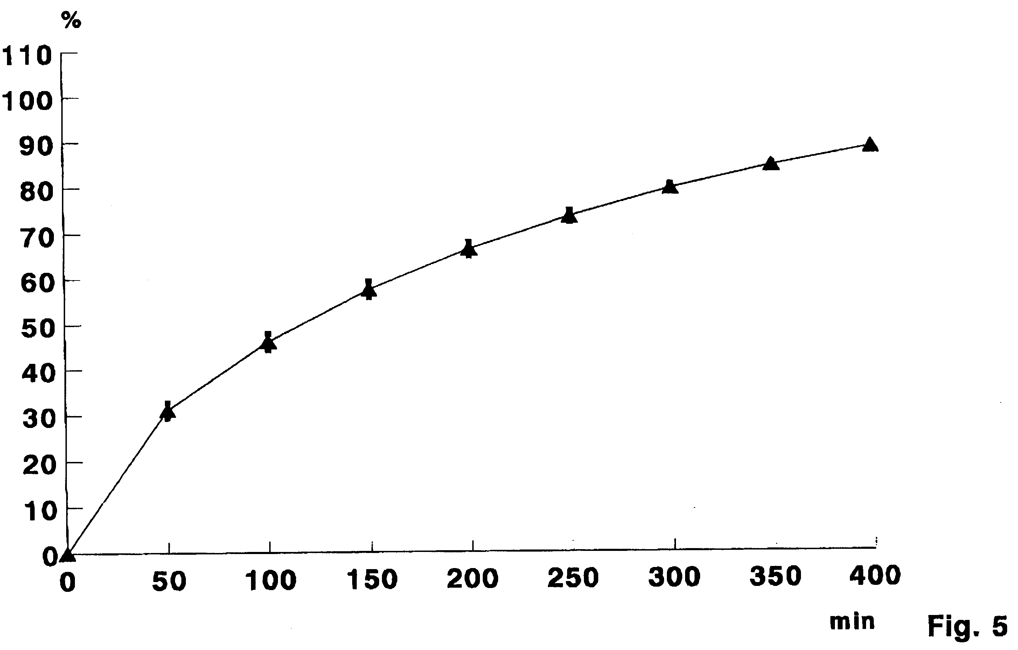

Die Resultate der in vitro Freisetzung der Pellets gemäss USP XXII (Phosphatpuffer, pH 7,2) in Fig. 5 zeigen das ungenügende Auflösungsverhalten dieser Pellets, obwohl der Anteil an Hilfsstoffen auf 25 % erhöht wurde.

### Vergleichsbeispiel F

| | |
|---|---|
| S(+)-Ibuprofen | 75,00 |
| Mikrokristalline Cellulose | 15,00 |
| PEG 4000 | 10,00 |
| Aqua purificata | q.s. |

Der Wirkstoff und die Hilfsmittel werden gemischt. Diese Mischung wird im Diosnamischer oder im Rotorprozessor auf übliche Weise solange mit Aqua purificata besprüht, bis sich Pellets der gewünschten Grösse bilden. Die Pellets werden dann getrocknet und mit dem Taumelsieb fraktioniert.

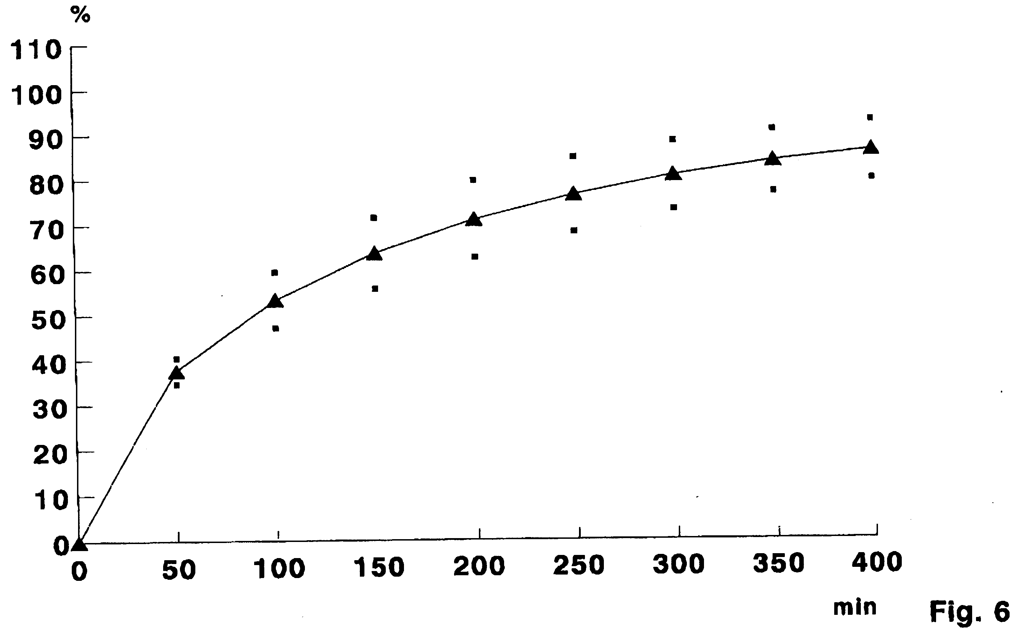

Die Resultate der in vitro Freisetzung der Pellets gemäss USP XXII (Phosphatpuffer, pH 7,2) in Fig. 6 zeigen das ungenügende Auflösungsverhalten dieser Pellets, obwohl der Anteil an Hilfsstoffen auf 25 % erhöht wurde.

### Beispiel 1

| | |
|---|---|
| S(+)-Ibuprofen | 95,0 |
| Natriumcarbonat | 2,0 |
| HPMC | 3,0 |
| Aqua purificata | q.s. |

S(+)-Ibuprofen, Natriumcarbonat und HPMC werden gemischt. Diese Mischung wird im Diosnamischer oder im Rotorprozessor auf übliche Weise solange mit Aqua purificata besprüht, bis sich Pellets der gewünschten Grösse bilden. Die Pellets werden dann getrocknet und mit dem Taumelsieb fraktioniert.

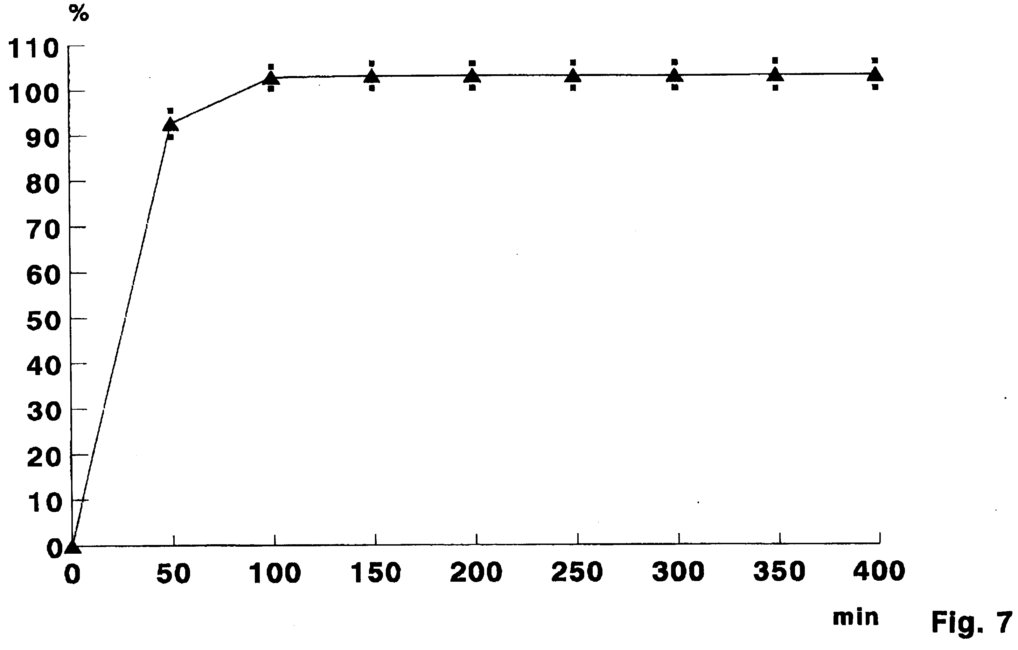

Mit dieser Formulierung gelang der Durchbruch zu wesentlich beschleunigter Freisetzung der Pellets gemäss USP XXII (Phosphatpuffer, pH 7,2). In Fig. 7 erkennt man den wesentlich schnelleren Verlauf der Freisetzung bei nur 5% Hilfsstoffanteil.

### Beispiel 2

| | |
|---|---|
| S(+)-Ibuprofen | 97,0 |
| Siliciumdioxid | 1,0 |
| Natriumcarbonat | 2,0 |
| Aqua purificata | q.s. |

S(+)-Ibuprofen, Siliciumdioxid und Natriumcarbonat werden gemischt. Diese Mischung wird im Diosnamischer oder im Rotorprozessor auf übliche Weise solange mit Aqua purificata besprüht, bis sich Pellets der gewünschten Grösse bilden. Die Pellets werden dann getrocknet und mit dem Taumelsieb fraktioniert.

Mit dieser Formulierung können bei nur 3% Hilfsstoffanteil über 90% an S(+)-Ibuprofen gemäss USP XXII (Phosphatpuffer, pH 7,2) freigesetzt werden.

### Beispiel 3

| | |
|---|---|
| S(+)-Ibuprofen | 90,0 |
| Mikrokristalline Cellulose | 4,0 |
| Lactose | 4,0 |
| Kaliumhydroxid 10% | 20,0 |
| Aqua purificata | q.s |

S(+)-Ibuprofen, Mikrokristalline Cellulose und Lactose werden gemischt. Diese Mischung wird im Diosnamischer oder im Rotorprozessor solange mit Kaliumhydroxid und anschliessend mit Aqua purificata besprüht, bis sich Pellets der gewünschten Grösse bilden. Die Pellets werden getrocknet und mit dem Taumelsieb fraktioniert.

### Beispiel 4

| | |
|---|---|
| S(+)-Ibuprofen | 92,0 |
| Mikrokristalline Cellulose | 5,0 |
| HPMC | 1,0 |
| Dinatriumhydrogenphosphat | 3,0 |
| Aqua purificata | q.s |

S(+)-Ibuprofen, Mikrokristalline Cellulose, HPMC und Dinatriumhydrogenphosphat werden gemischt. Diese Mischung wird im Diosnamischer oder im Rotorprozessor solange mit Aqua purificata besprüht, bis sich Pellets der gewünschten Grösse bilden. Die Pellets werden getrocknet und mit dem Taumelsieb fraktioniert.

### Beispiel 5 (magensaftresistentes Coating)

| | |
|---|---|
| S(+)-Ibuprofen Pellets | 100,0 |
| Aqua purificata | 12,3 |
| Antischaumemulsion SE2 | 0,04 |
| Siliciumdioxid | 3,0 |
| Diethylphthalat | 1,0 |
| Eudragit® L 30 D (LTS = Lacktrockensubstanz) | 25,0 LTS = 11,25% |

Die Antischaumemulsion und Siliciumdioxid werden in Aqua purificata suspendiert und anschliessend langsam zusammen mit Diethylphthalat ins Eudragit eingerührt. Die Pellets werden im Wirbelschichtgranulator (WSG) mit der Suspension besprüht.

### Beispiel 6 (Schutzcoating)

| | |
|---|---|
| S(+)-Ibuprofen Pellets | 100,0 |
| HPMC | 2,0 |
| Talcum | 2,0 |
| Aqua purificata | 22,0 LTS = 4,0% |

HPMC und Talcum werden in Aqua purificata suspendiert. Die Pellets werden im WSG mit dieser Suspension besprüht.

### Beispiel 7 (retardierende Pellets)

| | |
|---|---|
| S(+)-Ibuprofen Pellets | 100,0 |
| Aqua purificata | 26,0 |
| Antischaumemulsion SE2 | 0,02 |
| Talcum | 3,0 |
| Diethylphthalat | 1,0 |
| Eudragit® RS 30 D | 20,0 LTS = 10,0% |

Talcum und Antischaumemulsion werden in Aqua purificata suspendiert. Diese Suspension und Diethylphthalat werden sodann in Eudragit eingerührt. Darauf werden die Pellets im WSG mit obiger Suspension besprüht.

### Beispiel 8 (Angaben pro Tablette in mg)

| | |
|---|---|
| S(+)-Ibuprofen Pellets gecoatet (93,2% WS) | 429,0 |
| Mikrokristalline Cellulose | 160,0 |
| Maisstärke | 40,0 |
| Kaliumchlorid | 15,0 |
| PVP | 14,0 |
| Magnesiumstearat | 6,0 |
| Siliciumdioxid (WS = Wirkstoff) | 14,0 |

Die Mischung wird direkt zu Tabletten verpresst. Die Tabletten zerfallen innerhalb von 2 Minuten (gemäss USP XXII, S.1577) und zeigen eine Wirkstofffreigabe von über 90% freigesetztem S(+)-Ibuprofen innerhalb von 20 Minuten gemäss USP XXII, S.1578 (Phosphatpuffer, pH 7,2).

Als Alternativen zu Kaliumchlorid oder auch kumulativ können die folgenden Hilfsmittel verwendet werden:

Natriumchlorid, Calciumchlorid, Natriumcitrat, Kaliumhydrogenphosphat, Kaliumadipat, Calciumlactat, Kaliumcitrat.

Für Tabletten mit 50 mg, 100 mg, 200 mg, 600 mg und 800 mg berechnen sich die Komponenten proportional zu den obigen Angaben.

### Beispiel 9 (Angaben pro Tablette in mg)

| | |
|---|---|
| S(+)-Ibuprofen Pellets gecoatet (93.2% WS) | 429,0 |
| Emcompress | 140,0 |
| Ac-Di-Sol | 50,0 |
| HPMC | 20,0 |
| Cutina HR | 8,0 |
| Siliciumdioxid | 10,0 |

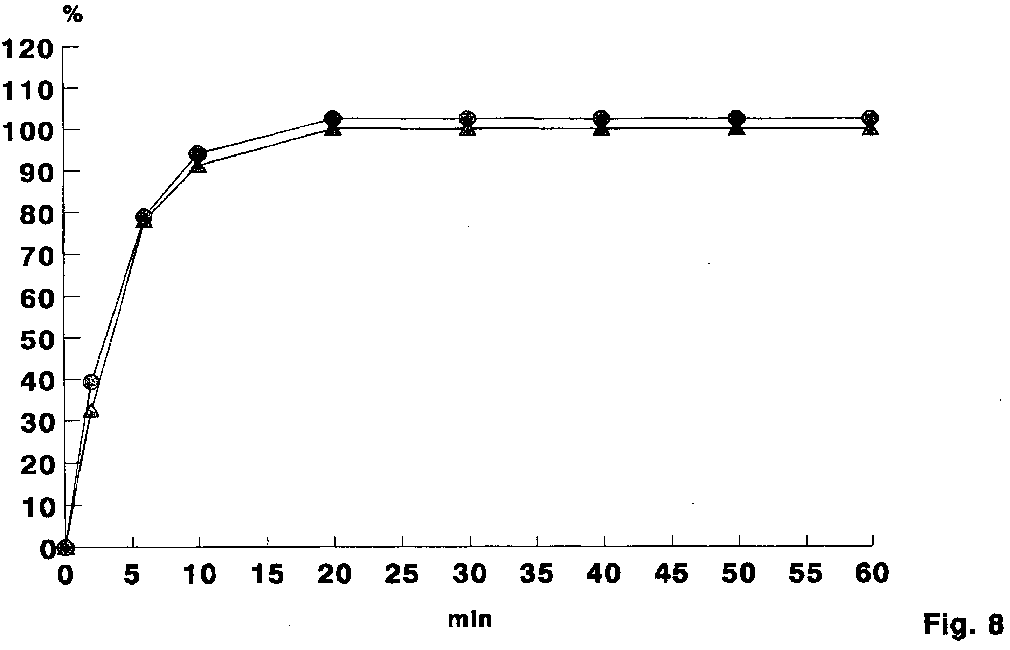

Die Mischung wird auf herkömmliche Art direkt zu Tabletten verpresst. Die Wirkstofffreisetzung beträgt beim Test mit 2 verschiedenen Chargen ebenfalls >90% in weniger als 20 Minuten gemäss USP XXII (Phosphatpuffer, pH 7,2)(Fig. 8).

### Beispiel 10 (Pellets in Kapseln gefüllt)

| | |
|---|---|
| S(+)-Ibuprofen Pellets (ungecoatet, 97% WS) | 413,0 |

Die gemäss Beispiel 2 hergestellten Pellets werden über eine Kapselfüllmaschine in Hartgelatinekapseln der Grösse 0 gefüllt.

Die Wirkstofffreisetzung aus diesen Pellets unter den vorgehend genannten Bedingungen (USP XXII, Phosphatpuffer, pH 7,2) ergibt ebenfalls Werte für S( +)-Ibuprofen >90% innerhalb von 20 Minuten.

### Beispiel 11

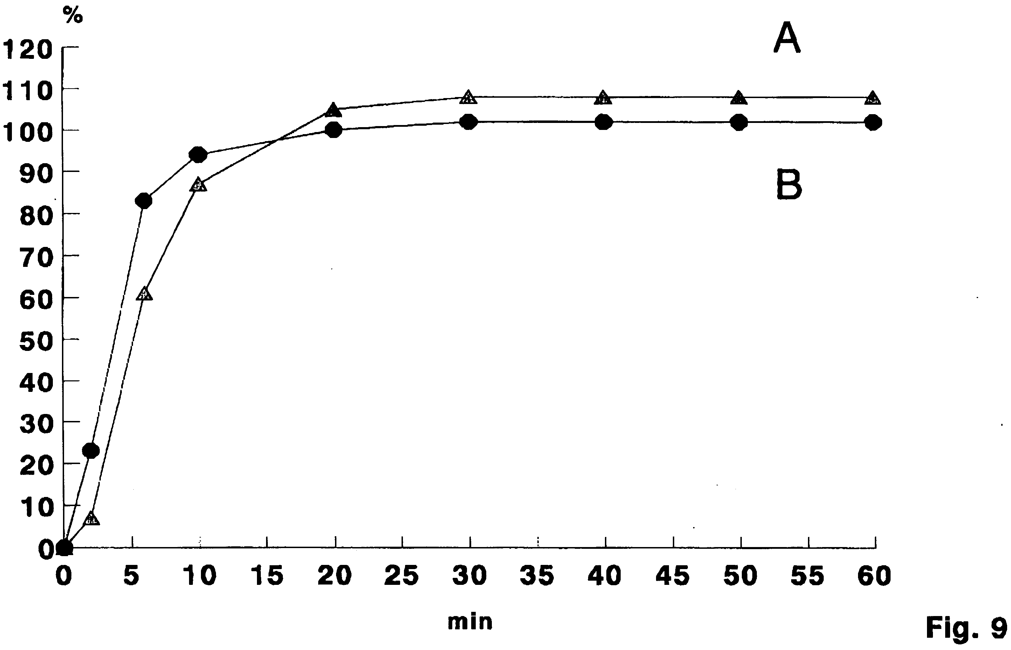

Die in vitro Freisetzung gemäss USP XXII (Phosphatpuffer, pH 7,2) von erfindungsgemässen Tabletten, die nach Beispiel 8 hergestellt wurden, wobei die Pellets dazu nach Beispiel 2 hergestellt und analog zu Beispiel 5 gecoatet worden waren, ist in Fig. 9-11 graphisch dargestellt.

Das Coating wies dabei folgende Zusammensetzung auf:

| | |
|---|---|
| S(+)-Ibuprofen | 100,0 |
| Aqua purificata | 18,9 |
| Antischaumemulsion SE2 | 0,04 |
| Siliciumdioxid | 0,4 |
| Titandioxid | 0,4 |
| Eudragit® L 30 D | 10,0 |

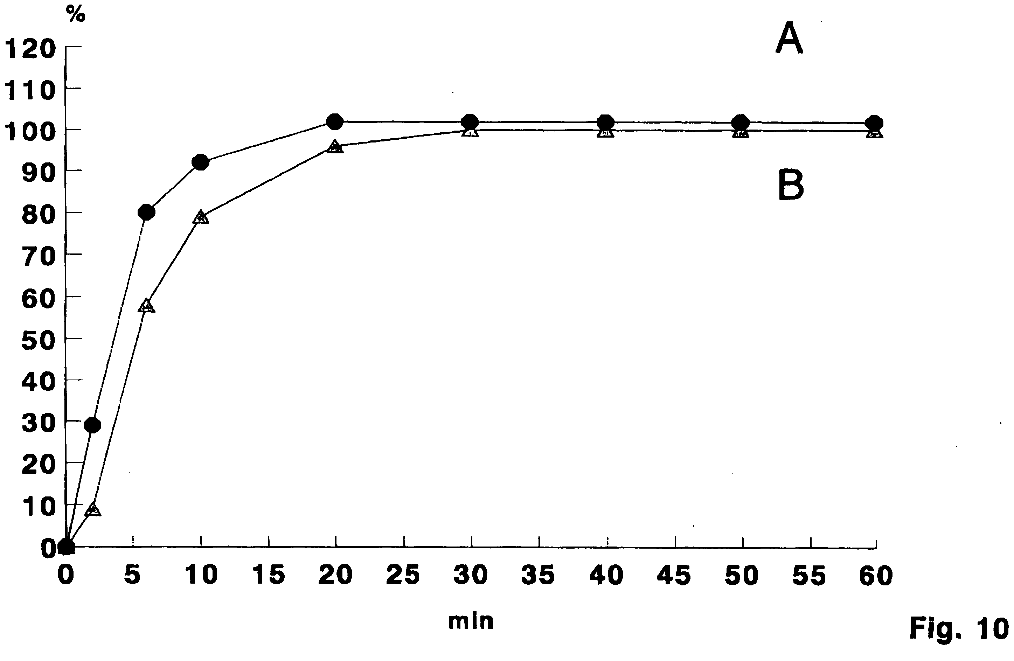

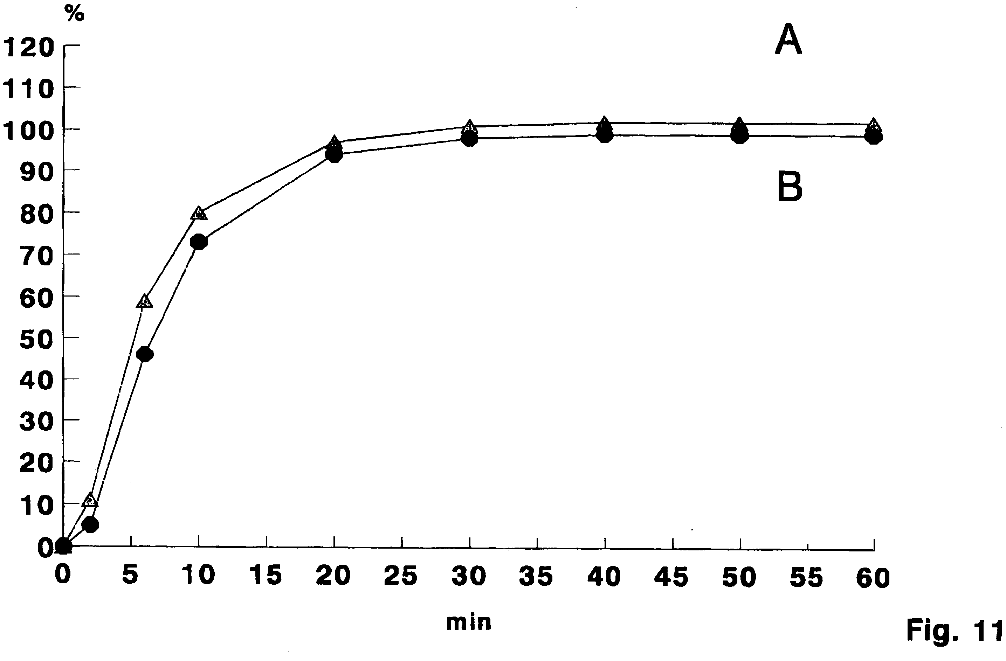

In Fig. 9 ist dabei die Freisetzung von 200 mg Tabletten gerade nach der Herstellung (Kurve A) und 3 Monate später (Lagerung bei 25°C und 60% relativer Feuchte; Kurve B) dargestellt. In Fig. 10 und 11 ist dies jeweils für die 400 mg und 600 mg Tabletten dargestellt.

Aus den Kurven ist u.a. ersichtlich, dass sich die Freisetzung des Wirkstoffs mit der Lagerung nicht signifikant verändert.

### Beispiel 12

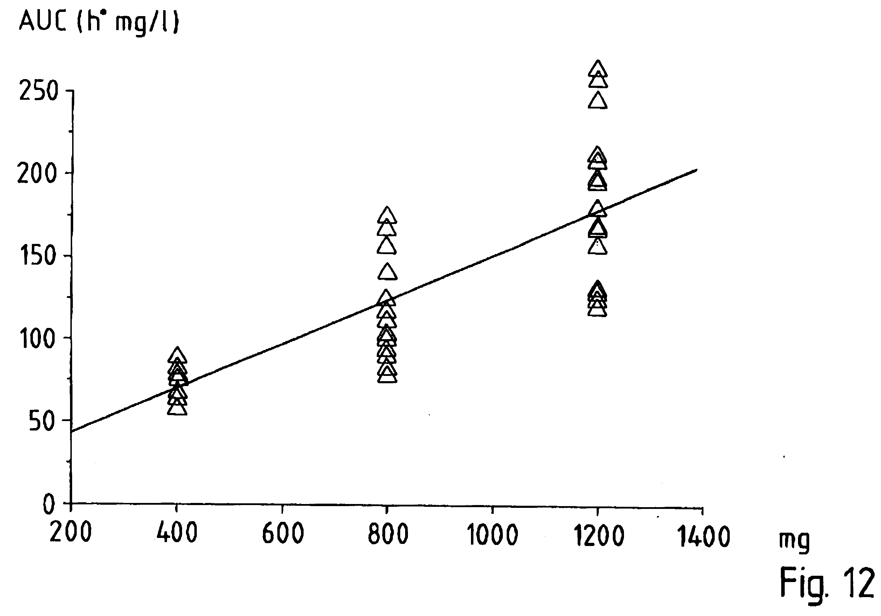

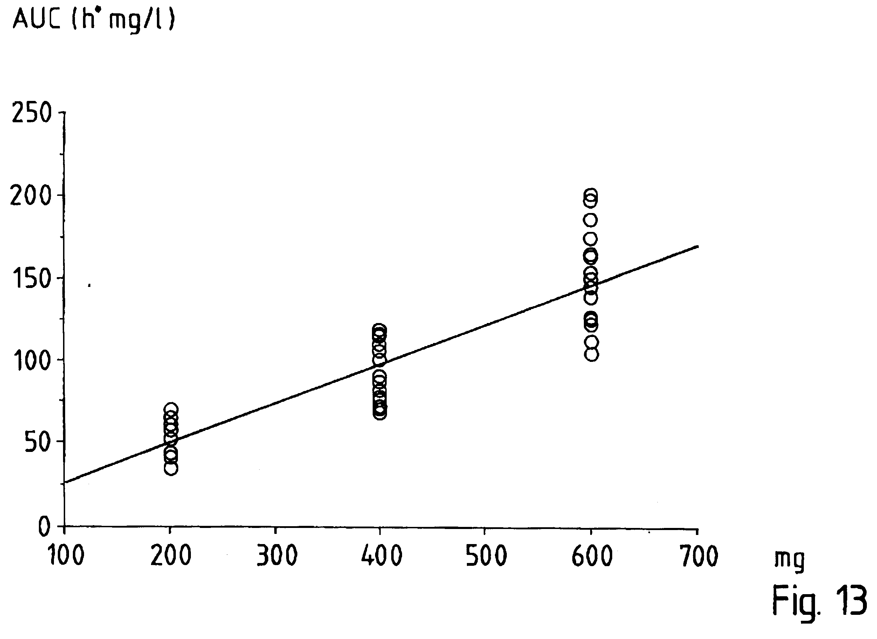

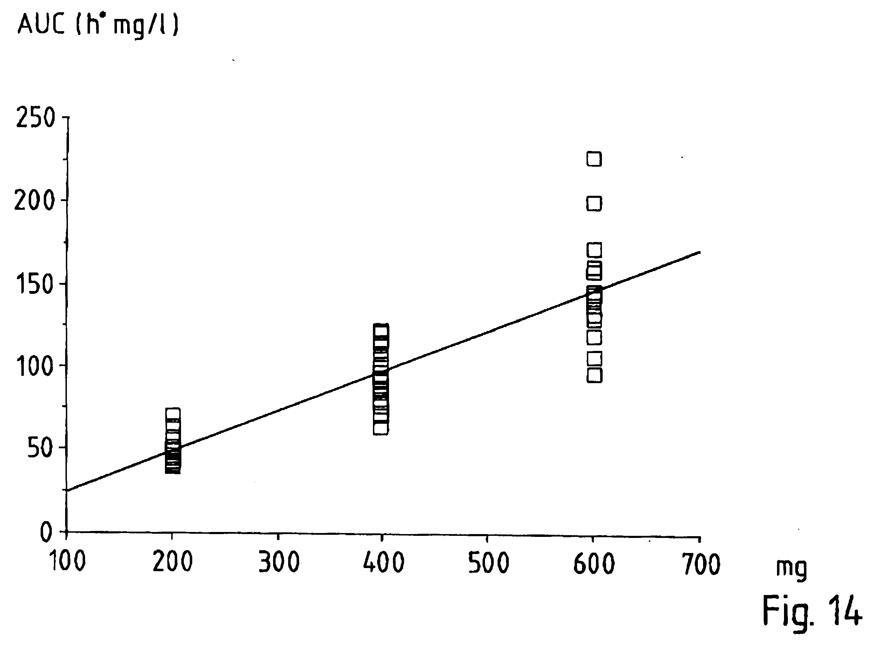

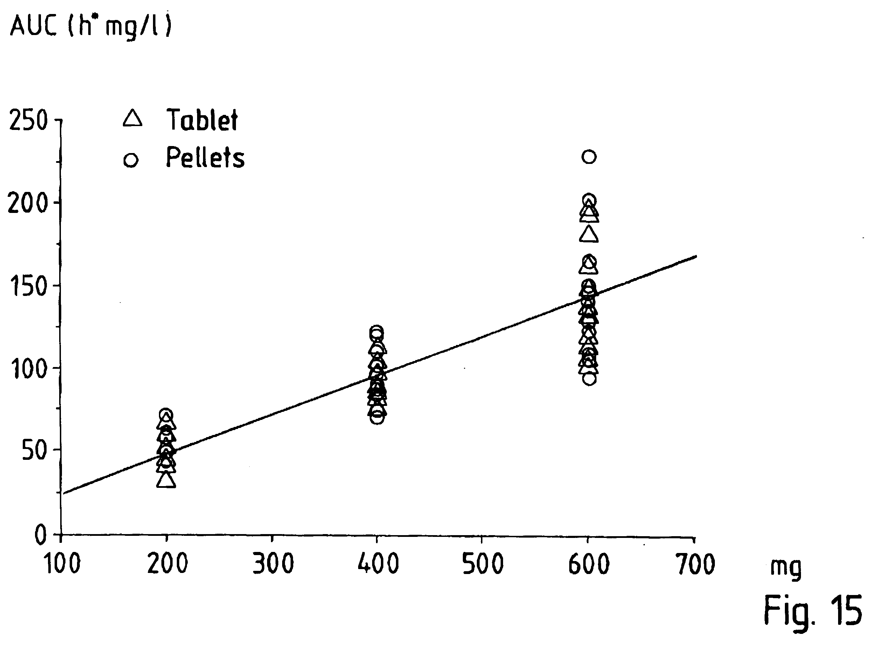

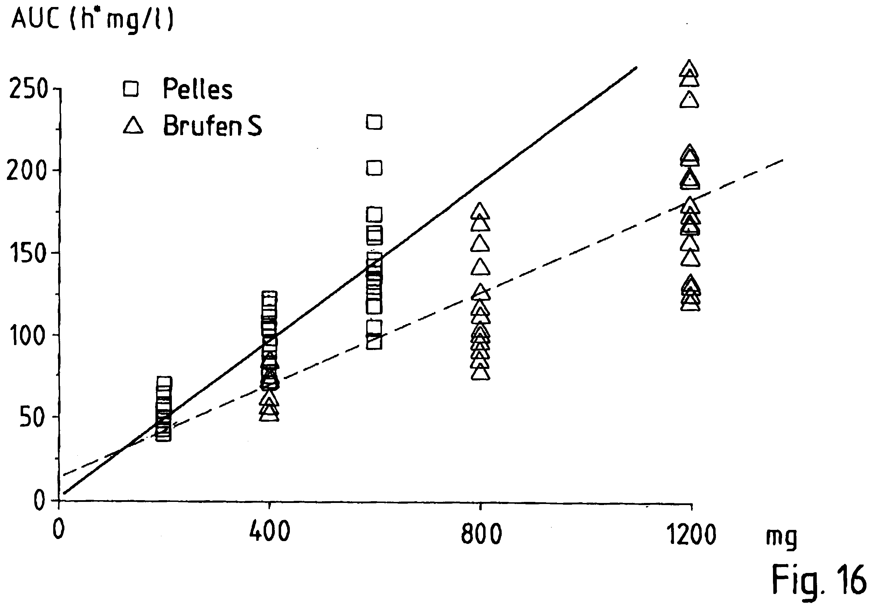

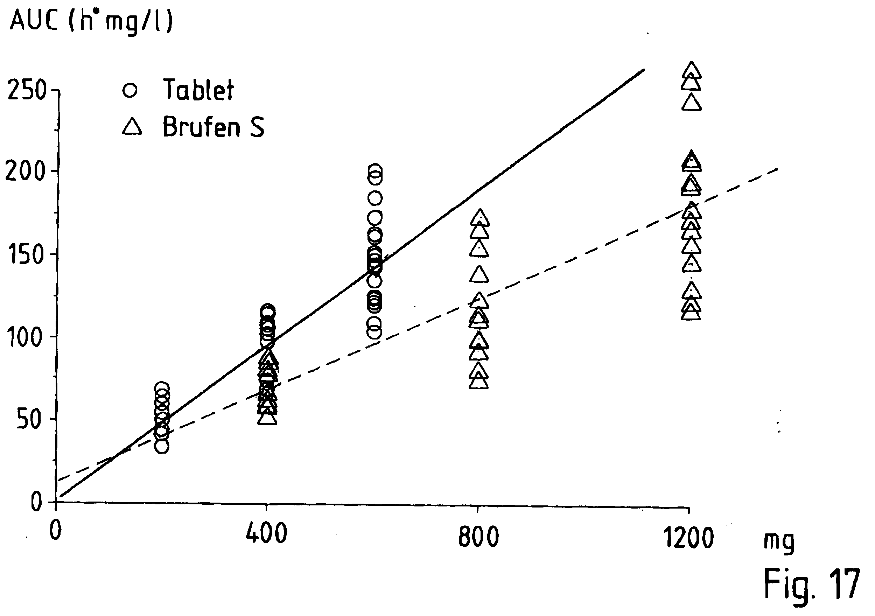

Die Bioverfügbarkeit von S(+)-Ibuprofen in Form der erfindungsgemässen Pellets in Kapseln mit 200 mg und der erfindungsgemässen Pellets in Tablettenform mit 200 mg, 400 mg und 600 mg wurde gegenüber Ibuprofen-Racemat in Form der im Markt erhältlichen Brufen Dragées mit 400 mg, 800 mg und 1200 mg verglichen. Die Prüfung erfolgte an 18 Testpersonen, die an 3 verschiedenen Tagen entweder 1, 2 oder 3 Kapseln mit Pellets à 200 mg oder je eine Tablette à 200 mg, 400 mg oder 600 mg S(+)-Ibuprofen oder je 1, 2 oder 3 Dragées à 400 mg Ibuprofen-Racemat erhielten. Die Resultate sind in Fig. 12-17 dargestellt.

Dabei zeigt Fig. 12 den Mittelwert des Verhältnisses der gemessenen S(+)-Ibuprofen-Mengen im Plasma der Testpersonen (als Flächen unter der Plasma-Zeit-Konzentrationskurven wiedergegeben = AUC in h*mg/l) mit der Dosis des Racemats. Fig. 13 zeigt den Mittelwert des Verhältnisses für die erfindungsgemässen Pellets in Tablettenform und Fig. 14 für die erfindungsgemässen Pellets in Kapseln. In jedem Fall nimmt die im Plasma gefundene Menge an S(+)-Ibuprofen mit der verabreichten Dosierung zu. Fig. 15 zeigt, dass die erfindungsgemässen Pellets in Tablettenform und in Kapseln im geprüften Dosisbereich bioäquivalent sind. Fig. 16 vergleicht das S(+)-Ibuprofen im Plasma nach Verabreichung der erfindungsgemässen Pellets in Kapseln und nach Verabreichung von Brufen-Dragées, die das Racemat enthalten, und Fig. 17 macht denselben Vergleich für erfindungsgemässe Pellets in Tablettenform. Mit der Regressionsanalyse wurden folgende Verhältnisse errechnet (Mittelwerte und 95%-Vertrauensbereich) :

| | |
|---|---|
| - erfindungsgemässe Tablette/Dragée: | 0,68 (0,62-0,73) |
| - erfindungsgemässe Kapsel/Dragée: | 0,67 (0,61-0,73) |

Bei Verwendung von erfindungsgemässen Pellets in Tablettenform oder in Kapseln kann die Dosierung gegenüber dem Racemat zur Erzielung gleicher Blutspiegelwerte somit um ca. ein Drittel erniedrigt werden. Dieses günstige Resultat wird durch Verwendung von S(+)-Ibuprofen in Kombination mit der erfindungsgemässen Galenik mit schneller Freisetzungscharakteristik erhalten.

## Patentansprüche

1. S(+)-Ibuprofenpellets, enthaltend 90,0-99,0 Gew.-% S(+)-Ibuprofen, und - im wesentlichen in Abwesenheit von Calciumsalzen - 0,1-10,0 Gew.-% Natriumcarbonat und/oder Dinatriumhydrogenphosphat und/oder Kaliumcarbonat.

2. Pellets nach Anspruch 1, enthaltend 96,0-98,0 Gew.-%, insbesondere 97 Gew.-%, S(+)-Ibuprofen und 1,0-3,0 Gew.-%, insbesondere 2,0 Gew-% Natriumcarbonat und/oder Dinatriumhydrogenphosphat und/oder Kaliumcarbonat.

3. Pellets nach einem der Ansprüche 1 oder 2, enthaltend 0,1 -5,0 Gew.-%, insbesondere 2,0 Gew.-% mindestens eines Hilfsstoffs.

4. Pellets nach Anspruch 3, enthaltend als Hilfsstoff Mikrokristalline Cellulose, Lactose, Hydroxypropylmethylcellulose und/oder vorzugsweise Siliciumdioxid.

5. Pellets nach einem der Ansprüche 1-4, enthaltend 90,0 -99,0 Gew.-%, insbesondere 97 Gew.-%, S(+)-Ibuprofen, 0,1 -5,0 Gew.-%, vorzugsweise 1 Gew.-%, Siliciumdioxid und 0,1 -10,0 Gew.-%, insbesondere 2,0 Gew.-%, Natriumcarbonat.

6. Mit einem Schutzcoating versehene Pellets nach einem der Ansprüche 1-5, enthaltend 0,1-10,0 Gew.-%, vorzugsweise 2,0 Gew.-%, eines Schutzcoatings, insbesondere Hydroxypropylmethylcellulose, Hydroxypropylcellulose, Polyvinylpyrrolidon, Polyvinylacetat, Methylcellulose oder Gemische davon.

7. Mit einem Schutzcoating versehene Pellets nach einem der Ansprüche 1-5, enthaltend 0,1-20,0 Gew.-%, vorzugsweise 10,0 Gew.-%, einer Methacrylsäure und/oder eines Ethylacrylats, wie Eudragit® L 30 D.

8. Mit einem magensaftresisten Coating versehene Pellets nach einem der Ansprüche 1-7, enthaltend 0,1-60,0 Gew.-%, vorzugsweise 25,0 Gew.-%, Eudragit® L 30 D, Hydroxypropylmethylcellulose oder Celluloseacetatphthalat oder Gemische davon.

9. Mit einem retardierenden Coating versehene Pellets nach einem der Ansprüche 1-7, enthaltend 0,1-50,0 Gew.-%, vorzugsweise 20,0 Gew.-%, Eudragit® NE 30 D, Eudragit® RS 30 D, Eudragit® RL 30 D oder Ethylcellulose oder Gemische davon.

10. Pellets nach Anspruch 6 oder 7, enthaltend als Schutzcoating eine Kombination aus Hydroxypropylmethylcellulose und Talcum oder Eudragit® L 30 D und Talcum, Titandioxid und/oder Siliciumdioxid und gegebenenfalls Antischaumemulsion, Diethylphthalat und/oder Polyethylenglykol.

11. Pellets nach Anspruch 8, enthaltend als magensaftresistentes Coating eine Kombination aus Eudragit® L 30 D, Diethylphthalat, Siliciumdioxid und gegebenenfalls Antischaumemulsion SE2.

12. Pellets nach Anspruch 9, enthaltend als retardierendes Coating Eudragit® RS 30 D, Talcum, Diethylphthalat und gegebenenfalls Antischaumemulsion SE2.

13. Pellets nach Anspruch 6 oder 7, enthaltend im Falle der Verwendung von Hydroxypropylmethylcellulose als Schutzcoating 0,1-8,0 Gew.-%, insbesondere 2,0 Gew.-%, Talcum, im Falle der Verwendung von Eudragit® L 30 D als Schutzcoating eine Kombination von Hilfsmitteln von jeweils 0,1-8,0 Gew.-%, insbesondere 0,4 Gew.-%, Siliciumdioxid und Titandioxid, und 0,1-8,0 Gew. -%, insbesondere 0,2 Gew.-%, Polyethylenglykol.

14. Pellets nach Anspruch 11, enthaltend eine Kombination von Hilfsmitteln von 0,1-8,0 Gew.-%, insbesondere 3,0 Gew. -%, Siliciumdioxid und 0,1-8,0 Gew.-%, insbesondere 1,0 Gew.-%, Diethylphthalat.

15. Pellets nach Anspruch 12, enthaltend eine Kombination von Hilfsmitteln von 0,1-8,0 Gew.-%, insbesondere 3,0 Gew. -%, Talcum und 0,1-8,0 Gew.-%, insbesondere 1,0 Gew. -%, Diethylphthalat.

16. Tabletten, enthaltend gecoatete Pellets nach einem der Ansprüche 1-15, enthaltend auf 400,0 mg S(+)-Ibuprofen 73,0 -410,0 mg, vorzugsweise 240,0-260,0 mg, besonders bevorzugt 250,0 mg, mindestens eines Tablettierhilfsmittels.

17. Tabletten nach Anspruch 16, enthaltend als Tabletttierhilfsmittel Mikrokristalline Cellulose, Calciumhydrogenphosphat, Lactose, Maisstärke, Crosscarmellose Natrium, Natriumstärkeglykolat, Polyvinylpyrrolidon, Hydroxypropylmethylcellulose, Magnesiumstearat, gehärtetes Rizinusöl, Siliciumdioxid, SiO₂, Kaliumchlorid, Calciumchlorid, Natriumchlorid, Natriumcitrat, Kaliumadipat, Calciumlactat, und/oder Kaliumcitrat.

18. Tabletten nach einem der Ansprüche 16 oder 17, enthaltend eine Kombination aus Tablettierhilfsmitteln, bestehend aus Mikrokristalliner Cellulose, Maisstärke, Polyvinylpyrrolidon, Magnesiumstearat, Siliciumdioxid und Kaliumchlorid.

19. Tabletten nach einem der Ansprüche 16-18, enthaltend 50-800 mg, vorzugsweise 100-400 mg, S(+)-Ibuprofen-Pellets nach einem der Ansprüche 1-9, und 15-50%, vorzugsweise 40%, Mikrokristalline Cellulose, 2,5-20%, vorzugsweise 10%, Maisstärke, 0,25-10%, vorzugsweise 3,5%, Polyvinylpyrrolidon, 0,25-5%, vorzugsweise 1,5%, Magnesiumstearat, 0,25 -7,5%, vorzugsweise 3,5%, Siliciumdioxid und 0,025-10%, vorzugsweise 3,75%, Kaliumchlorid.

20. Verfahren zur Herstellung von Pellets nach einem der Ansprüche 1-15, dadurch gekennzeichnet, dass man S( +)-Ibuprofen mit dem jeweiligen Natrium- oder Kaliumsalz und mindestens einem Hilfsstoff mischt und die Mischung solange mit einer gegebenenfalls basischen wässrigen Lösung, vorzugsweise Wasser, in einem Diosnamischer resp. in einem Rotorprozessor besprüht, bis sich Pellets der gewünschten Grösse bilden, diese dann trocknet, gegebenenfalls fraktioniert und gegebenenfalls mit einem Coating gemäss einem der Ansprüche 7-9 im Wirbelschichtgranulator (WSG) besprüht.

21. Verwendung der gecoateten Pellets nach einem der Ansprüche 1-15 zur Herstellung von Tabletten nach einem der Ansprüche 16-19, dadurch gekennzeichnet, dass man die gecoateten Pellets mit mindestens einem Tablettierhilfsmittel direkt zu Tabletten verpresst.

## Claims

1. S(+)-Ibuprofen pellets, containing 90,0-99,0% by weight S(+)-Ibuprofen, and - substantially in the absence of calcium salts - 0,1-10,0% by weight sodium carbonate and/or Disodiumhydrogenphosphate and/or potassium carbonate.

2. Pellets according to claim 1, containing 96,0-98,0% by weight, especially 97% by weight, S(+)-Ibuprofen and 1,0-3,0% by weight, especially 2,0% by weight sodium carbonate and/or Disodiumhydrogenphosphate and/or potassium carbonate.

3. Pellets according to one of the claims 1 or 2, containing 0,1-5,0% by weight, especially 2,0% by weight of at least one auxiliary material.

4. Pellets according to claim 3, containing as an auxiliary material microcrystalline cellulose, lactose, hydroxypropylmethylcellulose and/or preferably silicon dioxide.

5. Pellets according to any one of the claims 1-4, containing 90,0 -99,0% by weight, especially 97% by weight, S(+)-Ibuprofen, 0,1 -5,0% by weight, preferably 1% by weight, silicon dioxide and 0,1-10,0% by weight, especially 2,0% by weight, sodium carbonate.

6. Pellets provided with a protective coating according to any one of the claims 1-5, containing 0,1-10,0% by weight, preferably 2,0% by weight, of a protective coating, especially hydroxypropylmethylcellulose, hydroxypropyl-cellulose, polyvinylpyrrolidone, polyvinylacetate, methylcellulose or mixtures thereof.

7. Pellets provided with a protective coating according to any one of the claims 1-5, containing 0,1-20,0% by weight, preferably 10,0% by weight, of a methacrylic acid and/or of an ethyl acrylate, such as Eudragit® L 30 D.

8. Pellets provided with a gastric juice resistant coating according to any one of the claims 1-7, containing 0,1-60,0% by weight, preferably 25,0% by weight, Eudragit® L 30 D, hydroxypropylmethylcellulose or celluloseacetatephthalate or mixtures thereof.

9. Pellets provided with a retardant coating according to any one of the claims 1-7, containing 0,1-50,0% by weight, preferably 20,0% by weight, Eudragit® NE 30 D, Eudragit® RS 30 D, Eudragit® RL 30 D or ethylcellulose or mixtures thereof.

10. Pellets according to any one of the claims 6 or 7, containing as a protective coating a combination of hydroxypropylmethylcellulose and talcum or Eudragit® L 30 D and talcum, titanium dioxide and/or silicon dioxide and optionally an anti-foam emulsion, diethyl phthalate and/or polyethylene glycol.

11. Pellets according to claim 8, containing as a gastric juice resistant coating a combination of Eudragit® L 30 D, diethyl phthalate, silicon dioxide and optionally anti-foam emulsion SE2.

12. Pellets according to claim 9, containing as a retardant coating Eudragit® RS 30 D, talcum, diethyl phthalate and optionally anti-foam emulsion SE2.

13. Pellets according to claim 6 or 7 containing, in case of using hydroxypropylmethylcellulose as a protective coating, 0,1-8,0% by weight, especially 2,0% by weight, talcum, in case of using Eudragit® L 30 D as protective coating, a combination of auxiliary agents, of each time 0,1-8,0% by weight, especially 0,4% by weight, silicon dioxide and titanium dioxide, and 0,1-8,0% by weight, especially 0,2% by weight, polyethylene glycol.

14. Pellets according to claim 11, containing a combination of auxiliary agents of 0,1-8,0% by weight, especially 3,0% by weight, silicon dioxide and 0,1-8,0% by weight, especially 1,0% by weight, diethyl phthalate.

15. Pellets according to claim 12, containing a combination of auxiliary agents of 0,1-8,0% by weight, especially 3,0% by weight, talcum and 0,1-8,0% by weight, especially 1,0% by weight, diethyl phthalate.

16. Tablets, containing coated pellets according to any one of the claims 1-15, containing per 400,0 mg S(+)-Ibuprofen 73,0-410,0 mg, preferably 240,0-260,0 mg, especially preferred 250,0 mg, of at least one tabletting agent.

17. Tablets according to claim 16, containing as a tabletting agent microcrystalline cellulose, calcium hydrogenphosphate, lactose, maize starch, crosscarmellose sodium, sodium starch glycolate, polyvinyl pyrrolidone, hydroxypropylmethylcellulose, magnesium stearate, hardened castor oil, silicon dioxide, SiO₂, potassium chloride, calcium chloride, sodium chloride, sodium citrate, potassium adipate, calcium lactate, and/or potassium citrate.

18. Tablets according to any one of the claims 16 or 17, containing a combination of tabletting agents, consisting of microcrystalline cellulose, maize starch, polyvinylpyrrolidone, magnesium stearate, silicon dioxide and potassium chloride.

19. Tablets according to any one of the claims 16-18, containing 50-800 mg, preferably 100-400 mg, S(+)-Ibuprofen-pellets according to any one of the claims 1-9, and 15-50%, preferably 40%, microcrystalline cellulose, 2,5-20%, preferably 10%, maize starch, 0,25-10%, preferably 3,5%, polyvinylpyrrolidone, 0,25-5%, preferably 1,5%, magnesium stearate, 0,25-7,5%, preferably 3,5%, silicon dioxide and 0,025-10%, preferably 3,75%, potassium chloride.

20. Process for the production of pellets according to any one of the claims 1-15, characterised in mixing S(+)Ibuprofen with the respective sodium or potassium salt and at least one auxiliary agent and spaying the mixture in a Diosnamixer resp. in a Rotorprocessor with an optionally basic aqueous solution, preferably water, for a time long enough until pellets of the desired size have formed, subsequently drying, optionally fractionating and optionally spraying the pellets in a fluidised bed granulator (WSG) with a coating according to any one of the claims 7-9.

21. Use of the coated pellets according to any one of the claims 1-15 for the production of tablets according to any one of the claims 16-19, characterised in that the coated pellets together with at least one tabletting auxiliary agent are pressed directly into tablets.

## Revendications

1. Des pellets de S(+)-ibuprophène, contenant 90.0-99.0 % en poids de S(+)-ibuprophène, et - essentiellement en absence de sels de calcium - 0.1-10.0 % en poids de carbonate de sodium et/ ou de disodium-hydrogène-phosphate et/ ou de carbonate de potassium.

2. Des pellets selon la revendication 1, contenant 96.0-98.0 % en poids, particulièrement 97 % en poids, de S(+)-ibuprophène, et 1.0-3.0 % en poids, particulièrement 2.0 % en poids, de carbonate de sodium et/ ou de disodiumhydrogène-phosphate et/ ou de carbonate de potassium.

3. Des pellets selon la revendication 1 ou 2, contenant 0.1-5.0 % en poids, particulièrement 2.0 % en poids, d'au moins un adjuvant.

4. Des pellets selon la revendication 3, contenant comme adjuvant de la cellulose microcristalline, lactose, hydroxypropylméthylcellulose et/ ou de préférence dioxyde de silicium.

5. Des pellets selon l'une quelconque des revendications 1 à 4, contenant 90.0-99.0 % en poids, particulièrement 97 % en poids, de S(+)-ibuprophène, 0.1-5.0 % en poids, de préférence 1 % en poids, de dioxyde de silicium et 0.1-10.0 % en poids, particulièrement 2.0 % en poids, de carbonate de sodium.

6. Des pellets avec une couche de protection selon l'une quelconque des revendications 1 à 5, contenant 0.1-10.0 % en poids, de préférence 2.0 % en poids, d'une couche de protection, particulièrement hydroxypropylméthylcellulose, hydroxypropylcellulose, polyvinylpyrrolidone, polyvinylacétate, méthylcellulose ou leurs mélanges.

7. Des pellets avec une couche de protection selon l'une quelconque des revendications 1 à 5, contenant 0.1-20.0 % en poids, de préférence 10.0 % en poids, d'un acide méthacrylique et/ ou d'un éthylacrylate, tel qu'Eudragit® L 30 D.

8. Des pellets avec une couche résistante au suc gastrique selon l'une quelconque des revendications 1 à 7, contenant 0.1-60.0 % en poids, de préférence 25.0 % en poids d'Eudragit® L 30 D, hydroxypropylméthylcellulose ou acétylphtalate de cellulose ou leurs mélanges.

9. Des pellets avec une couche retardante selon l'une quelconque des revendications 1 à 7, contenant 0.1-50.0 % en poids, de préférence 20.0 % en poids, d'Eudragit® NE 30 D, Eudragit® RS 30 D, Eudragit® RL 30 D ou éthylcellulose ou leurs mélanges.

10. Des pellets selon la revendication 6 ou 7, contenant comme couche de protection une combinaison d'hydroxypropylméthyl-cellulose et talc ou Eudragit® L 30 D et talc, dioxyde de titane et/ ou dioxyde de silicium et optionnellement de l'émulsion anti-mousse, diéthylphtalate et/ ou polyéthylène-glycol.

11. Des pellets selon la revendication 8, contenant comme couche résistante au suc gastrique une combinaison d'Eudragit® L 30 D, diéthylphtalate, dioxyde de silicium et optionnellement de l'émulsion anti-mousse SE2.

12. Des pellets selon la revendication 9, contenant comme couche retardante de l'Eudragit® RS 30 D, talc, diéthylphtalate et optionnellement de l'émulsion anti-mousse SE2.

13. Des pellets selon la revendication 6 ou 7, contenant, dans le cas où on utilise de l'hydroxypropylméthylcellulose comme couche de protection, 0.1-8.0 % en poids, particulièrement 2.0 % en poids, de talc, dans le cas où on utilise de l'Eudragit® L 30 D comme couche de protection, une combinaison d'adjuvants, à chaque fois de 0.1-8.0 % en poids, particulièrement 0.4 % en poids, de dioxyde de silicium et dioxyde de titane, et 0.1-8.0 % en poids, particulièrement 0.2 % en poids, de polyéthylène-glycol.

14. Des pellets selon la revendication 11, contenant une combinaison d'adjuvants de 0.1-8.0 % en poids, particulièrement de 3.0 % en poids, de dioxyde de silicium et 0.1-8.0 % en poids, particulièrement 1.0 % en poids, de diéthylphtalate.

15. Des pellets selon la revendication 12, contenant une combinaison d'adjuvants de 0.1-8.0 % en poids, particulièrement de 3.0 % en poids, de talc et 0.1-8.0 % en poids, particulièrement 1.0 % en poids, de diéthylphtalate.

16. Des comprimés, contenant des pellets enrobés selon l'une quelconque des revendications 1 à 15, contenant pour 400 mg de S(+)-ibuprophène 73.0-410.0 mg, de préférence 240.0-260.0 mg, particulièrement préféré 250.0 mg, d'au moins un adjuvant pour la formation de comprimés.

17. Des comprimés selon la revendication 16, contenant comme adjuvant pour la formation des comprimés de la cellulose microcristalline, calcium-hydrogène-phosphate, lactose, amidon de maïs, crosscarmellose de sodium, glycolate d'amidon de sodium, polyvinylpyrrolidone, hydroxypropylméthylcellulose, stéarate de magnésium, huile de ricin durci, dioxyde de silicium, SiO₂, chlorure de potassium, chlorure de calcium, chlorure de sodium, citrate de sodium, adipate de potassium, lactate de calcium et/ ou citrate de potassium.

18. Des comprimés selon la revendication 16 ou 17, contenant une combinaison d'adjuvants pour la formation des comprimés, comprenant cellulose microcristalline, amidon de maïs, polyvinylpyrrolidone, stéarate de magnésium, dioxyde de silicium et chlorure de potassium.

19. Des comprimés selon l'une quelconque des revendications 16 à 18, contenant 50-800 mg, de préférence 100-400 mg, de pellets de S(+)-ibuprophène selon l'une quelconque de revendications 1 à 9, et 15-50%, de préférence 40 %, de cellulose microcristalline, 2.5-20 %, de préférence 10 %, d'amidon de maïs, 0.25-10 %, de préférence 3.5 %, de polyvinylpyrrolidone, 0.25-5 %, de préférence 1.5 %, de stéarate de magnésium, 0.25-7.5 %, de préférence 3.5 %, de dioxyde de silicium et 0.025-10 %, de préférence 3.75 %, de chlorure de potassium.

20. Un procédé pour la fabrication de pellets selon l'une quelconque des revendications 1 à 15, caractérisé en ce qu'on mélange du S(+)-ibuprophène avec le sel de sodium ou de potassium respectif et au moins un adjuvant et en ce qu'on arrose le mélange avec une solution aqueuse optionnellement basique, de préférence de l'eau, dans un Diosna-mélangeur respectivement un Rotor-processeur, pendant un temps assez long jusqu'à la formation de pellets de la taille souhaitée, puis leur séchage, optionnellement leur fractionnement et optionnellement leur arrosage dans un granulateur à couche fluidifiée (WSG) avec une couche selon l'une quelconque des revendications 7 à 9.

21. Utilisation des pellets enrobés selon l'une quelconque des revendication 1 à 15 pour la fabrication de comprimés selon l'une quelconque des revendications 16-19, caractérisée en ce qu'on comprime les pellets enrobés avec au moins un adjuvant pour la formation des comprimés directement en des comprimés.
